# EUROPEAN PATENT SPECIFICATION

(11) **EP 2 726 445 B1**
(45) Date of publication and mention of the grant of the patent: **22.05.2019**
(21) Application number: 12737905.5
(22) Date of filing: 28.06.2012
(51) Int. Cl.: C07C 2/76, C07C 15/02, C07C 15/27, B01J 37/08, B01J 29/44, B01J 29/068, B01J 37/18

(54) **METHOD AND CATALYST FOR THE ALKYLATION OF AROMATIC COMPOUNDS WITH ALKANES**
VERFAHREN UND KATALYSATOR ZUR ALKYLIERUNG AROMATISCHER VERBINDUNGEN MIT ALKANEN
PROCÉDÉ ET CATALYSEUR POUR L'ALKYLATION DES COMPOSÉS AROMATIQUES AVEC DES ALCANES

(30) Priority: 29.06.2011 EP 11171909
(43) Date of publication of application: 07.05.2014
(73) Proprietor: Stamicarbon B.V. acting under the name of MT Innovation Center, 6135 KW Sittard (NL); Universität Stuttgart, 70174 Stuttgart (DE)
(72) Inventor: Traa, Yvonne, 6135 KW Sittard (NL); Geiss, Daniel, 6135 KW Sittard (NL)
(74) Representative: V.O.
(86) International application number: PCT/NL2012/050455
(87) International publication number: WO 2013/002638

(56) References cited:
- EP-A1- 0 250 879
- DE-A1-102006 059 800
- SEALY S ET AL: "Direct alkylation of toluene with ethane on bifunctional zeolite catalysts", APPLIED CATALYSIS A: GENERAL, ELSEVIER SCIENCE, AMSTERDAM, NL, vol. 294, no. 2, 10 October 2005 (2005-10-10), pages 273-278, XP025332924, ISSN: 0926-860X, DOI: 10.1016/J.APCATA.2005.07.042 [retrieved on 2005-10-10]
- BRESSEL ET AL: "Influence of aluminum content, crystallinity and crystallite size of zeolite Pd/H-ZSM-5 on the catalytic performance in the dehydroalkylation of toluene with ethane", MICROPOROUS AND MESOPOROUS MATERIALS, ELSEVIER SCIENCE PUBLISHING, NEW YORK, US, vol. 109, no. 1-3, 21 December 2007 (2007-12-21), pages 278-286, XP022399279, ISSN: 1387-1811, DOI: 10.1016/J.MICROMESO.2007.05.002
- BISCARDI J A ET AL: "Structure and Density of Active Zn Species in Zn/H-ZSM5 Propane Aromatization Catalysts", JOURNAL OF CATALYSIS, ACADEMIC PRESS, DULUTH, MN, US, vol. 179, no. 1, 1 October 1998 (1998-10-01), pages 192-202, XP004447324, ISSN: 0021-9517, DOI: 10.1006/JCAT.1998.2177

## Description

### Field of the invention

The invention pertains to a method for the alkylation of aromatic compounds with alkanes. Particularly, the invention relates to the direct alkylation of aromatic hydrocarbons with short-chain alkanes, having a chain length of from 1 to 12 carbon atoms.

### Background of the invention

Alkylated aromatics, e.g. ethylbenzene and ethyltoluene, find widespread usage. In conventional processes to produce such alkyl aromatics, an aromatic hydrocarbon is alkylated with a reactive agent such as olefin, alkyl halide or alkyl alcohol. Processes for the direct alkylation of aromatics with alkanes are virtually non-existent. Yet, this would be desired since regular alkylation agents, such as alkenes, are expensive. It would be desired for the alkylation of aromatics to be possible with alkanes instead of alkenes because alkanes directly occur in nature in the form of natural gas, whereas alkenes have to be made from alkanes. Thus, alkanes are cheaper than alkenes, and a process step can be saved. However, to be able to use alkanes as alkylating agents, a very active and selective catalyst is needed since the reaction is severely limited by thermodynamics.

An existing process is the "M-Forming" process. This starts with longer alkanes, cracks them and uses then the olefinic fragments again as alkylating agents for aromatics alkylation. Similarly, US 4,899,008 refers to a direct catalytic alkylation of mononuclear aromatics with lower alkanes. Therein an acid H-ZSM-5 catalyst is used. The main alkylation products are not direct alkylation products but products formed from cracked propane. Since cracking of propane produces methane and ethene, it is likely that ethene acted as alkylating agent.

A reference on the direct alkylation of aromatics with alkanes is WO 99/59942. The reaction is catalyzed by a molecular sieve catalyst comprising incorporated metal. Herein a hydrocarbon feed containing an aromatic hydrocarbon is contacted with an alkane of at least 15 carbon atoms

Sealy S., Applied Catalysis A, 294(2005), 273-278 as well Bressel A, Microporous and Mesoporous Materials, 109(2008), 278-286 disclose a process for the alkylation of toluene with ethane in presence of a catalyst containing palladium supported on ZSM-5 zeolite.

DE102006059800 discloses a process for the alkylation of benzene with dodecane using a catalyst containing platinum and zinc supported on beta zeolite.

Reactions conditions for the conversion of such longer alkanes, however, are not normally suitable for light alkanes. The problem with longer alkanes is their high reactivity, particularly towards cracking. The problem with light alkanes, such as those having chain lengths of from 1 to 12 carbon atoms, and more particularly from 1 to 8 carbon atoms, is that they are difficult to activate.

Hence, a demand exists in the art to provide a more versatile process for the direct alkylation of aromatic compounds, which would enable both light and heavy alkanes to be employed. Also, it is desired to improve yield.

### Summary of the invention

In order to better address one or more of the foregoing desires, the invention presents, in one aspect, a process for the alkylation of an aromatic compound, comprising contacting the aromatic compound with an alkane under elevated temperature, in the presence of a catalyst composition comprising a catalytically active metal and a promoter metal on ZSM-5 zeolite wherein the catalytically active metal is palladium, and the promoter is zinc.

In another aspect, the invention provides the use of a catalyst composition as defined above, for the activation of an alkane towards the direct alkylation of an aromatic compound.

### Brief description of the drawings

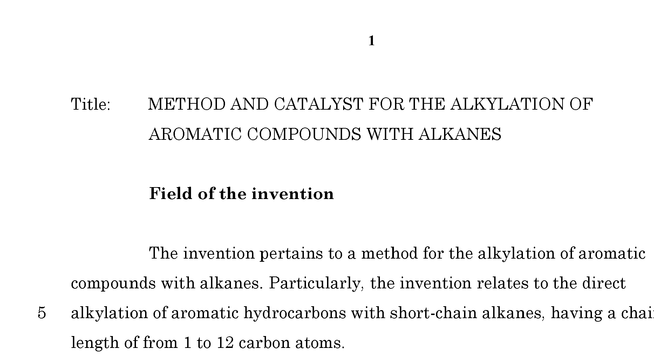

Fig. 1 is a graph representing the yield of ethyltoluenes over time, upon direct alkylation of toluene with ethane. Depicted is the result of a process under the influence of three catalyst compositions of the invention. The measurement points hereof are represented by black, white and gray bullets. The graph includes a comparison with a catalyst composition not according to the invention. The measurement points hereof are indicated with black and white triangles.

### Detailed description of the invention

In a broad sense, the invention is based on the judicious insight that a palladium catalyst in combination with zinc as a promoter, is able to achieve the activation of alkanes towards the direct alkylation of aromatic compounds. The combination of the catalyst and the promoter is presented on a porous support, which is ZSM-5 zeolite.

The zeolite-type support is desired for the presence of acidic sites. Amongst known zeolites, ZSM-5 and the like are suitable to prevent coking and to suppress thermodynamically favored reactions. The spaciousness index and the modified constraint index are known methods to characterize zeolites and zeolite-type materials. These terms are well-defined in the art. Reference can be made, *inter alia,* to the "Handbook of Porous Solids", F. Schüth, K.S.W. Sing, J. Weitkamp (eds.), Wiley-VCH, 2002. Particularly for zeolites, see, e.g., pages 699, for SAPOs, e.g., pp. 815, for MOFs, e.g., pp. 1190, and for spaciousness index and modified constraint index e.g., pp. 1015.

The support material desirably has acidic sites. On this basis, good results can be obtained with medium Si/Al molar ratios. However, for the optimal working of the promoter, it is believed that reasonable ion exchange capacities are desired, which would imply reasonably low Si/Al molar ratios. All in all, it is preferred for the zeolites to have Si/Al molar ratios between 2 and 100, preferably between 5 and 50, more preferably between 10 and 35, most preferably between 15 and 30.

The molar ratio of zinc to palladium generally is between 0.01 and 5, preferably between 0.1 and 1.5, most preferably between 0.1 and 0.5.

The catalyst composition of the invention generally comprise 0.1 wt.% to 5 wt.% of palladium, preferably 0.2 wt.% to 1 wt. %, most preferably between 0.4 wt.% and 0.9 wt.%. With the addition of zinc as a promoter, the content of the mainly active metal can be reduced.

The catalyst composition of the invention serves to activate alkanes towards the direct alkylation of aromatic compounds.

Light alkanes, as used in the present invention, are aliphatic hydrocarbons having chain lengths of 1 to 12 carbon atoms, preferably and more particularly from 1 to 8 carbon atoms, more preferably from 1 to 6 carbon atoms. These alkanes can be linear or branched, with n-alkanes being preferred. Still more preferred alkanes have chain lengths of 2 to 4 carbon atoms. Ethane and propane are the most preferred. With light alkanes, and particularly with ethane and propane, a particular challenge has been overcome by presenting a catalyst composition that is actually suitable to support a direct alkylation reaction of aromatic compounds.

The source of the alkanes used in the alkylation reaction is not of particular relevance. E.g., the process of the invention can also be carried out using light alkanes that are formed from prior cracking of higher alkanes. However, it will be understood that in order to fully enjoy the benefits of the invention, it is preferred to employ light alkanes provided from direct, existing sources of such alkanes.

The catalyst comprising palladium and zinc not only presents the aforementioned advantages in the alkylation of aromatic with light alkanes, but also is advantageous for use in the alkylation of aromatics with higher alkanes, i.e. of more than 12 carbon atoms, particularly 15 or more. These alkanes may range from a linear or very slightly branched paraffin having from 15 to 22 carbon atoms, to light, medium or heavy slack wax, paraffinic FCC bottoms, deasphalted hydrocracked bottoms, Fischer-Tropsch synthetic distillate and wax, deoiled wax or polyethylene wax, light or heavy cycle oil. Other sources include waxy shale oil, tar sands and synthetic fuels.

Aromatic compounds to be alkylated by the process of the present invention preferably comprise one to three phenyl rings. Other rings, such as five-membered or seven-membered rings fused into an aromatic ring system are conceivably also alkylated by the process of the invention. The aromatic compounds can comprise full carbon rings, but also heterocyclic aromatic compounds are included. Preferred aromatic compounds are selected from the group consisting of benzene, toluene, other alkyl aromatics, phenol, anthracene, phenanthrene, and pyridine..

In the process of the invention, as in largely any catalytic alkylation process, temperature, and preferably also pressure, will be elevated as compared to room temperature. Preferably, the reaction is conducted at a temperature of 200°C to 500 °C, more preferably 320°C to 400°C.

The pressure employed will generally depend on the type of reactor used. Preferred pressures are within a range of from 1 bar to 200 bar, more preferably 5 bar to 50 bar, and most preferably 7 bar to 20 bar.

In the preferred embodiment of a combination of palladium and zinc, it is believed that zinc serves to dilute the palladium, and thus modifies the activity and selectivity of the catalyst into the direction desired for the direct alkylation of aromatic compounds.

Whilst similar catalysts may already have been used for other applications, e.g., the dehydrogenation of alkanes, this is not the case for the alkylation of aromatics with alkanes, particularly with light alkanes. The use of zinc allows considerably higher yields of the desired alkyl aromatics, i.e., about 12% instead of 5% during the alkylation of toluene with ethane in a fixed-bed reactor at 24 bar and 350°C (see Fig. 1).

The invention will further be described with respect to non-limiting examples and with reference to a figure. The invention is not limited thereto but only by the claims. Where the term "comprising" is used in the present description and claims, it does not exclude other elements or steps. Where an indefinite or definite article is used when referring to a singular noun, e.g., "a" or "an", "the", this includes a plural of that noun unless something else is specifically stated.

### Example 1

### Preparation of the catalyst

Palladium ion exchange was carried out by adding drop wise under stirring an aqueous solution of 0.304 g Pd(NH₃)₄Cl₂ (40.62 wt.-% Pd, ChemPur) in 250 ml demineralized water to a suspension of 9.446 g (dry mass) zeolite (Si/Al molar ratio of the zeolite is between 10 and 35) in 250 ml demineralized water. The mixture was stirred at room temperature for 24 hours, filtered and dried at 353 K for another 24 h.

It will be understood that the amounts of Pd salt, water and zeolite can be varied. It is also possible to save Pd salt by not filtering the solution but carefully evaporating the water.

The catalyst was then calcined at 823 K in nitrogen for another 24 h and cooled to room temperature. 2.613 g (dry mass) zeolite were suspended in 25 ml demineralized water and 0.013 g of zinc acetate (C₄H₆O₄Zn·2H₂O, Fluka 99.0%) were added. Then the water was carefully removed in a rotary evaporator, thereby impregnating the catalyst with the zinc salt. Afterwards, the catalyst was dried again at 353 K for 24 h.

### Example 2

### Catalytic Experiments

For the catalytic experiments, the zeolite powder was pressed without a binder, crushed and sieved to get a particle size between 0.2 and 0.3 mm. The catalyst was activated in situ, prior to starting the experiment. To achieve a high dispersion of the noble metal, 0.5 g of the catalyst were first heated in flowing synthetic air (150 cm³ min⁻¹) at a rate of 0.25 K min⁻¹ to a final temperature of 573 K, then it was switched to nitrogen (150 cm³ min⁻¹) and heated with a rate of 1.7 K min⁻¹ to a final temperature of 623 K. Afterwards the catalyst was reduced under a constant stream of hydrogen (150 cm³ min⁻¹) at 623 K for 4 h.

Catalytic experiments were performed in a flow-type apparatus with a fixed-bed reactor from stainless steel. Ethane (99.95 vol.-%, Westfalen AG) and nitrogen (99.999 vol.-%, Westfalen AG) were fed with an *ṅ*_{nitrogen} /*ṅ*ₑₜₕₐₙₑ ratio of approximately 4 through a toluene (> 99.9 %, Merck) saturator containing Chromosorb P-NAW (Macherey-Nagel). Nitrogen was used as an internal standard but also to ensure that a relatively low *ṅ*ₑₜₕₐₙₑ/*ṅ*ₜₒₗᵤₑₙₑ feed ratio of 5±1 could be achieved at the high pressure applied. The reaction was carried out at a total pressure of 24 bar and a reaction temperature of (350±2) °C. The WHSV (toluene and ethane) was 1.0 h-¹. Product analysis was achieved using an on-line sampling system, a capillary gas chromatograph and a CP-PoraPLOT Q column (length: 30 m, inner diameter: 0.32 mm, film thickness: 20 µm, Chrompack). Two detectors in series were employed, namely, a thermal conductivity detector followed by a flame ionization detector. Correction factors for the two detectors were determined separately. With ethane as tie substance, the results from both detectors were combined. From the mass and molar flows, the selectivities of all products were calculated in mol%. The yields were determined from the selectivities and the toluene conversion.

In Fig. 1 a graphic representation is given of the yield of ethyltoluenes during the alkylation of toluene with ethane on zeolite catalysts in a fixed-bed reactor (pressure: 24 bar; reaction temperature: 350 °C).

## Claims

1. A process for the alkylation of an aromatic compound, comprising contacting the aromatic compound with an alkane under elevated temperature, in the presence of a catalyst composition comprising a catalytically active metal and a promoter metal on a ZSM-5 zeolite support, wherein the catalytically active metal is palladium, and the promoter is zinc.

2. A process according to claim 1, wherein the catalyst composition comprises 0.1 wt.% to 5 wt.% of palladium.

3. A process according to claim 2, wherein the catalyst composition comprises 0.2 wt.% to 1 wt.% of palladium, preferably between 0.4 wt.% and 0.9 wt.%.

4. A process according to any one of the preceding claims, wherein the alkane is a light alkane having 1 to 12 carbon atoms, preferably 2 to 4 carbon atoms.

5. A process according to any one of the claims 1-4, wherein the alkane has more than 15 carbon atoms, preferably more than 22 carbon atoms.

6. A process according to any one of the preceding claims, wherein the aromatic compound is selected from the group consisting of benzene, toluene, phenol, anthracene, phenanthrene, and pyridine.

7. A process according to any one of the preceding claims, wherein the reaction is conducted at a temperature of 200°C to 500 °C, preferably 320°C to 400°C.

8. A process according to any one of the preceding claims, wherein the reaction is conducted under a pressure within a range of from 1 bar to 200 bar.

9. A process according to claim 8, wherein the pressure is 5 bar to 50 bar, preferably 7 bar to 20 bar.

10. The use of a catalyst composition as defined in any one of the claims 1 to 3, for the activation of an alkane towards the direct alkylation of an aromatic compound.

## Patentansprüche

1. Verfahren für die Alkylierung einer aromatischen Verbindung, umfassend das Inkontaktbringen der aromatischen Verbindung mit einem Alkan unter erhöhter Temperatur, in der Gegenwart einer Katalysatorzusammensetzung, umfassend ein katalytisch aktives Metall und ein Promotormetall auf einem ZSM-5-Zeolithträger, wobei das katalytisch aktive Metall Palladium ist, und der Promotor Zink ist.

2. Verfahren nach Anspruch 1, wobei die Katalysatorzusammensetzung 0,1 Gew.% bis 5 Gew.% Palladium umfasst.

3. Verfahren nach Anspruch 2, wobei die Katalysatorzusammensetzung 0,2 Gew.% bis 1 Gew.% Palladium umfasst, vorzugsweise zwischen 0,4 Gew.% und 0,9 Gew.%.

4. Verfahren nach einem der vorhergehenden Ansprüche, wobei das Alkan ein Leichtalkan ist mit 1 bis 12 Kohlenstoffatomen, vorzugsweise 2 bis 4 Kohlenstoffatomen.

5. Verfahren nach einem der Ansprüche 1-4, wobei das Alkan mehr als 15 Kohlenstoffatome hat, vorzugsweise mehr als 22 Kohlenstoffatome.

6. Verfahren nach einem der vorhergehenden Ansprüche, wobei die aromatische Verbindung ausgewählt ist aus der Gruppe, bestehend aus Benzol, Toluol, Phenol, Anthracen, Phenanthren, und Pyridin.

7. Verfahren nach einem der vorhergehenden Ansprüche, wobei die Reaktion bei einer Temperatur von 200°C bis 500 °C durchgeführt wird, vorzugsweise 320°C bis 400°C.

8. Verfahren nach einem der vorhergehenden Ansprüche, wobei die Reaktion unter einem Druck innerhalb eines Bereichs von 1 bar bis 200 bar durchgeführt wird.

9. Verfahren nach Anspruch 8, wobei der Druck 5 bar bis 50 bar ist, vorzugsweise 7 bar bis 20 bar.

10. Verwendung einer Katalysatorzusammensetzung wie definiert in einem der Ansprüche 1 bis 3, für die Aktivierung eines Alkans gegenüber der direkten Alkylierung einer aromatischen Verbindung.

## Revendications

1. Procédé pour l'alkylation d'un composé aromatique, comprenant la mise en contact du composé aromatique avec un alcane à une température élevée, en présence d'une composition de catalyseur comprenant un métal catalytiquement actif et un métal promoteur sur un support de zéolite ZSM-5, où le métal catalytiquement actif est le palladium et le promoteur est le zinc.

2. Procédé selon la revendication 1, où la composition de catalyseur comprend 0,1 % en poids à 5 % en poids de palladium.

3. Procédé selon la revendication 2, où la composition de catalyseur comprend 0,2 % en poids à 1 % en poids de palladium, de préférence entre 0,4 % en poids et 0,9 % en poids.

4. Procédé selon l'une quelconque des revendications précédentes, où l'alcane est un alcane léger ayant 1 à 12 atomes de carbone, de préférence 2 à 4 atomes de carbone.

5. Procédé selon l'une quelconque des revendications 1-4, où l'alcane a plus de 15 atomes de carbone, de préférence plus de 22 atomes de carbone.

6. Procédé selon l'une quelconque des revendications précédentes, où le composé aromatique est choisi dans le groupe consistant en le benzène, le toluène, le phénol, l'anthracène, le phénanthrène et la pyridine.

7. Procédé selon l'une quelconque des revendications précédentes, où la réaction est conduite à une température de 200°C à 500°C, de préférence de 320°C à 400°C.

8. Procédé selon l'une quelconque des revendications précédentes, où la réaction est conduite sous une pression dans une plage de 1 bar à 200 bar.

9. Procédé selon la revendication 8, où la pression est 5 bar à 50 bar, de préférence 7 bar à 20 bar.

10. Utilisation d'une composition de catalyseur telle que définie dans l'une quelconque des revendications 1 à 3, pour l'activation d'un alcane en direction de l'alkylation directe d'un composé aromatique.
